# EUROPEAN PATENT SPECIFICATION

(11) **EP 0 407 439 B1**
(45) Date of publication and mention of the grant of the patent: **28.04.1993**
(21) Application number: 89904124.8
(22) Date of filing: 23.03.1989
(51) Int. Cl.: B29B 17/00, B29B 13/10, B29B 9/14

(54) **A MOULDING COMPOUND AND A METHOD OF MANUFACTURING THE COMPOUND**
FORMMASSE UND VERFAHREN ZU IHRER HERSTELLUNG
COMPOSE DE MOULAGE ET PROCEDE DE FABRICATION D'UN TEL COMPOSE

(30) Priority: 23.03.1988 GB 8806915
(43) Date of publication of application: 16.01.1991
(73) Proprietor: CHAS. A. BLATCHFORD & SONS LIMITED, Basingstoke Hampshire RG22 4AH (GB)
(72) Inventor: EVANS, Andrew, John, Sear, Farnham Surrey GU10 4QQ (GB)
(74) Representative: Blatchford, William Michael
(86) International application number: GB8900313
(87) International publication number: WO8909123

(56) References cited:
- FR-A- 2 180 031
- Soviet Inventions Illustrated, section chemical, abstract 88-019434, Derwent Publications Ltd,(London, GB) & SU,A,1313824 (Lengd Mech Inst) 30.05.87
- Derwent Japanese Patents Report, section chemical,abstract 83-44965K, Derwent Publications Ltd, (London,GB) & JP,A,58053951 (Yutaka Light Kogyos) 30.03.83

## Description

This invention relates to a moulding compound which is a fibre and plastics composite and to a method of manufacturing the compound.

It is well known to mould composites containing a thermosetting plastics matrix material and reinforcing carbon, aramid or other fibres. The fibres may be used in the form of a woven cloth, fibre tows, or braid. To simplify the moulding process the materials of the composite are commonly supplied as a mixture of plastics and fibre in the proportions required for moulding, the plastics material being in a partly cured, tacky state. This impregnated mixture is generally known as "prepreg". Moulding begins with the cutting of the prepreg to suitable sizes and shapes depending on the size and shape of the article to be moulded, and is followed by the laying of the material in or on a mould. Moulding is performed by subjecting the material to heat, generally under pressure, yielding an article which has a considerably higher strength-to-weight ratio than most metals.

Prepreg materials, particularly those containing carbon or aramid fibres, are, however, expensive. In cutting such a material to size, a considerable quantity is wasted, particularly when the fibres are in the form of a cloth, since the offcuts are usually too small to be used in further mouldings. Often, the proportion of the original material represented by offcuts is as high as 25 per cent, and unless they can be used in making small components, they have conventionally been discarded as unusable scrap.

Russian Patent Application No. SU-A-1 313 824 discloses a method of re-using glass-fibre filled epoxide waste to manufacture a building article. In this method, the waste is cooled at a temperature of between -120 and -160 degrees celsius, crushed, mixed with expanded polystyrene granules, and layered in a mould with epoxide binder. When the mould is full, the article is hardened by heating.

According to a first aspect of this invention, a method of manufacturing a moulding compound which is a fibre and plastics composite includes the steps of providing a mixture of fibres impregnated with a plastics matrix material, and cooling the mixture to a temperature of less than or equal to -50 degrees celsius, characterised in that the plastics material is in the form of a flexible partly cured thermosetting plastics material, and in that, after cooling, the mixture is milled while it is still at a temperature of less than or equal to -50 degrees celsius to yield a compound having fibres which are shorter than those of the said provided mixture. The filament lengths of the compound are typically in the range of from 0.2mm to 10mm. Material so treated retains the original proportions of plastics and fibre materials and can, if required, be moulded to form articles which are subsequently isotropic, i.e. in which the fibres are to a large degree randomly orientated.

Preferably, the mixture is cooled using liquid nitrogen both prior to and during milling.

The ratio of fibre material to plastics matrix material in the yielded compound is preferably governed by and substantially corresponds to the fibre/plastics ratio of the starting mixture, which is typically scrap prepreg offcuts.

The milled compound is particularly suitable for producing articles by compression moulding, and, unlike anisotropic prepregs, possesses good flow characteristics and can be moulded around sharp corners. Since the proportions of fibre and plastics materials can be largely governed by and correspond to the proportions of the materials of the starting mixture, i.e. prior to milling, it is possible to achieve a higher fibre content than in a typical conventional dough moulding compound, giving a superior strength isotropic material. The starting material can be glass-fibre prepreg, but in general higher strength can be obtained using carbon or aramid as the reinforcing fibre material.

The method is primarily applicable to composites with a thermosetting plastics matrix such as epoxy resin or heat-curing polyester. The low temperatures used, as well as making the material relatively brittle for the milling process, prevent the onset of curing which can otherwise arise as a result of heat generated in the milling operation.

The invention also includes, according to a second aspect thereof, a moulding compound produced by the method referred to above and comprising a quantity of randomly oriented fragments each comprising a bundle of thermosetting plastics-coated fibre filaments with filament lengths in the range of from 0.2mm to 10mm, and with the plastics matrix material in a partly cured condition.

A fibre-reinforced plastics article may be moulded by placing in a mould a quantity of fragments each comprising a bundle of fibres coated with a thermosetting plastics material in a partly cured state and having filament lengths in the range of from 0.2mm to 10mm, and heating the mass of fragments in the mould under pressure to cure the plastics material, thereby yielding a substantially isotropic fibre-reinforced component.

The invention will now be described by way of example with reference to the drawings in which:-
Figure 1 is a block diagram illustrating the principle of a preferred method in accordance with the invention for producing a moulding compound;
Figure 2 is a diagram of apparatus for carrying out the method of Figure 1, including a rotating blade granulator;
Figure 3 is a diagrammatic cross-section of a pin-disc mill; and
Figure 4 is a diagrammatic cross-section of a mould assembly for moulding an article from a moulding compound produced in accordance with the invention.

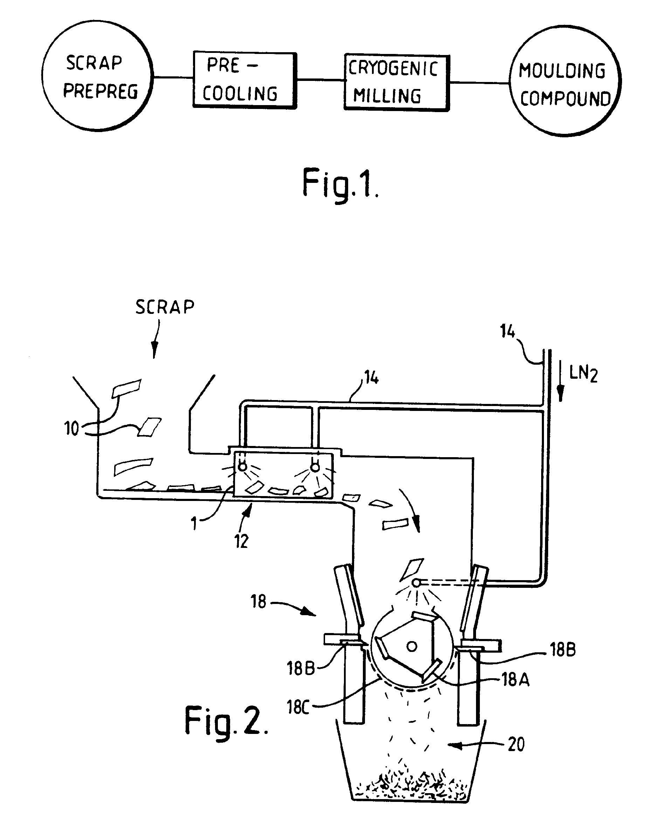

Referring to Figures 1 and 2, the starting material used for a process in accordance with the invention may be woven cloth, tows or braid of carbon or other fibre materials, impregnated with a thermosetting plastics material using, for example, a resin dipping technique. The applicants have in particular used scrap offcuts 10 of carbon fibre woven cloth impregnated with a 'B' stage epoxy resin matrix material. However, virgin material can be used providing it is cut into suitable pieces for the process to be described below. Care must be taken that the material used is within its shelf life.

After having had the release paper removed, the prepreg offcuts 10 are fed into a cryogenic pre-cooler 12 using liquid nitrogen as the cooling medium at -196°C, supplied via pipes 14. The offcuts are tumbled in a cooling vessel 16 whilst the liquid nitrogen is injected into the vessel. Once the charge has been sufficiently chilled, it is immediately transferred to a mill 18, which may be of the type having a plurality of cutting blades 18A formed as a rotatable assembly with the cutting edges of the blades defining a cylindrical surface centred on the axis of rotation. Such a mill is often referred to as a granulator. As they rotate, the blades successively engage stationary blades 18B mounted parallel to the axis of rotation with a shearing action. A mesh 18C with a predetermined aperture size is mounted beneath the blades to allow passage only of milled fragments 20 of less than a predetermined size. In this way the fibre lengths of the moulding compound are controlled to lie within a predetermined range.

In the preferred method in accordance with the invention the duration of the milling process and the mesh aperture size is arranged to yield fragments of impregnated fibre compound in which the filament length is mostly in the range of from 0.2mm to 6mm and preferably 1mm to 2mm.

By milling the material at a temperature less than -50°C, the tendency of substantial quantities of the material to adhere to the blades of the mill or for the plastics matrix to begin curing is largely avoided, and the effect on the blades of the abrasive nature of certain fibre materials such as carbon and aramid fibres can be substantially reduced due to the material being brittle at this low temperature. During the milling process the resin also protects to some extent the fibre surfaces, each filament being individually coated with plastic.

The resulting shredded compound retains the optimum ratio of fibre to plastics for maximum strength, and the fibres are "wetted" with the plastics material to the same extent as in the starting material; both of these requirements would be difficult to achieve if the fibres were coated after milling. However, if a higher resin content is required, for instance to improve the surface finish of the moulding, compatible resins may be added during the milling operation to distribute resin uniformly throughout the compound. As with conventional prepreg material the compound has a shelf life which depends on the storage temperature, and for this reason is best stored in a refrigerator.

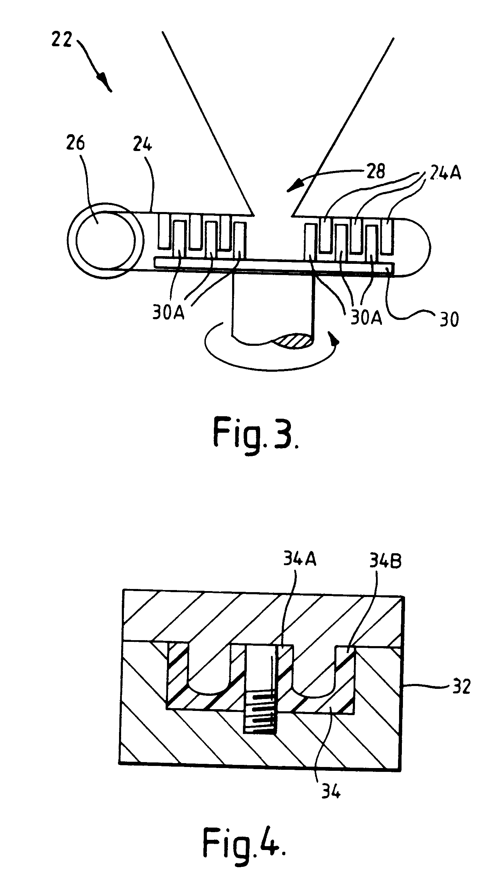

As an alternative to a rotating blade granulator as shown in Figure 2, the milling may be performed using a pin-disc mill as shown diagrammatically in Figure 3. The pin-disc mill 22 comprises a horizontal circular chamber 24 having a tangential outlet opening 26 and a central inlet opening 28 in the centre of the chamber. Within the chamber 24 is a horizontalrotatable disc 30 having rings of upstanding pins 30A arrangedaround the axis of rotation of the disc, and, extending downwardly from the top plate of the chamber, are further rings of pins 24A located at radii such that they are situated between and adjacent the pins 30A of the rotating disc 30. Material fed into the inlet 28 must pass between the relatively rotatingpins to reach the outlet 26, and in so doing is ground into fragments of a predetermined size.

In many moulding application a weighed charge of the compound may simply be poured into one half of a mould and then compressed and heated between two mould halves. In these circumstances the compound has been found to have good flow characteristics and to be very suitable for moulding around sharp corners, unlike cloth-based prepregs. When moulded, the compound is substantially isotropic in that the filaments are mostly randomly oriented.

The compound may be used to mould a variety of articles particularly articles of intricate shape, in which a high strength to weight ratio is needed. The applicants have used the compound to mould artificial limb components such as a component for mounting a knee joint mechanism on an artificial limb socket for an above knee amputee. A mould 32 for moulding the component is shown in section in Figure 4, the material 34 of the component being shown hatched. The component comprises a circular spacer about 10mm in thickness having a central boss 34A and outer flange 34B.

In summary, a moulding compound containing fibres coated with a thermosetting plastics material, is produced by providing a starting material comprising fibres of a filament length generally greater than 10mm, the fibres being pre-coated with the plastics material, cooling the starting material to a temperature below -50°C, and milling the material while it is still at low temperature to yield fragments in which the filaments length is in the range 0.2mm to 10mm, and preferably mostly in the range 1mm to 2mm.

The moulding compound comprises a mixture of carbon, aramid or other fibres having a filament length in the range 0.2mm to 10mm, preferably mostly in the range 1mm to 2mm, and a thermosetting plastics material. The plastics material forms a coat on the fibre filaments. The compound is preferably in the form of loose fragmentseach comprising a bundle of coated filaments, so that the fragments can be placed in a mould to form a moulded article in which the fibre filaments are substantially randomly oriented.

## Claims

1. A method of manufacturing a moulding compound which is a fibre and plastics composite, including the steps of: providing a mixture of fibres impregnated with a plastics matrix material, and cooling the mixture to a temperature of less than or equal to -50 degrees celsius, characterised in that the plastics material is in the form of a flexible partly cured thermosetting plastics material, and in that, after cooling, the mixture is milled while it is still at a temperature of less than or equal to -50 degrees celsius to yield a compound having fibres which are shorter than those of the said provided mixture.

2. A method according to claim 1, wherein the filament lengths of the milled compound are in the range of from 0.2mm to 10mm.

3. A method according to claim 1 or claim 2, wherein the fibres are carbon fibres.

4. A method according to claim 1 or claim 2, wherein the fibres are aramid fibres.

5. A method according to any preceding claim, wherein the mixture is woven or unidirectional prepreg cloth, tows or braid having fibres of an average filament length greater than 10mm.

6. A method according to any preceding claim, wherein the mixture is cooled using liquid nitrogen.

7. A method according to claim 6, wherein the mixture is cooled during milling using liquid nitrogen.

8. A method according to any preceding claim, wherein the milled compound comprises loose fragments each comprising a bundle of plastics coated filaments of an average filament length in the range of from 1 to 2mm.

9. A method according to any preceding claim, characterised in that the ratio of fibre material to plastics matrix material in the yielded compound is governed by and substantially corresponds to the fibre/plastics ratio of the material forming the starting mixture.

10. A method according to any preceding claim, characterised in that the material forming the starting mixture comprises scrap prepreg offcuts.

11. A moulding compound produced according to the method of any of the preceding claims, characterised by a quantity of randomly orientated fragments each comprising a bundle of thermosetting plastics-coated fibre filaments with filament lengths in the range of from 0.2mm to 10mm, and with the plastics matrix material in a partly cured condition.

12. A compound according to claim 11, characterised in that the fibre filaments are carbon or aramid fibre filaments.

13. A compound according to claim 11 or claim 12, characterised in that the average filament length of the fibre filaments is in the range of from 1mm to 2mm.

14. A method of moulding a fibre-reinforced plastics article comprising placing in a mould a moulding compound according to claim 11, claim 12 or claim 13, each fragment comprising a bundle of fibres coated with a thermosetting plastics material in a partly cured state, and heating the mass of fragments in the mould under pressure to cure the plastics material, thereby yielding a substantially isotropic fibre-reinforced component.

## Patentansprüche

1. Verfahren zur Herstellung einer Formmasse, bei der es sich um ein Faser-Plastikmassen(Kunststoff)-Verbundmaterial handelt, das die folgenden Stufen umfaßt:
Bereitstellen einer Mischung von Fasern, die mit einem Plastikmassen(Kunststoff)-Matrixmaterial imprägniert sind, und
Abkühlen der Mischung auf eine Temperatur von ≦ -50°C, dadurch gekennzeichnet, daß
das Plastik(Kunststoff)-Material in Form eines flexiblen, teilweise ausgehärteten, wärmehärtbaren Plastik(Kunststoff)-Materials vorliegt und daß
nach dem Abkühlen die Mischung, während sie noch eine Temperatur von ≦ -50°C hat, gemahlen wird unter Bildung einer Formmasse, die Fasern enthält, die kürzer sind als diejenigen der Ausgangsmischung.

2. Verfahren nach Anspruch 1, worin die Faserlängen der gemahlenen Mischung in dem Bereich von 0,2 bis 10 mm liegen.

3. Verfahren nach Anspruch 1 oder 2, worin die Fasern Kohlenstoffasern sind.

4. Verfahren nach Anspruch 1 oder 2, worin die Fasern Aramidfasern sind.

5. Verfahren nach irgendeinem vorhergehenden Anspruch, worin die Mischung ein gewebtes oder in einer Richtung Kunststoff-imprägniertes Gewebe, Werg oder Geflecht ist, das Fasern mit einer durchschnittlichen Faserlänge von mehr als 10 mm aufweist.

6. Verfahren nach irgendeinem vorhergehenden Anspruch, worin die Mischung unter Verwendung von flüssigem Stickstoff abgekühlt wird.

7. Verfahren nach Anspruch 6, worin die Mischung während des Mahlens unter Verwendung von flüssigem Stickstoff gekühlt wird.

8. Verfahren nach irgendeinem vorherhergehenden Anspruch, worin die gemahlene Mischung lose (nicht zusammenhängende) Fragmente umfaßt, die jeweils ein Bündel von Plastik(Kunststoff)-beschichteten Fasern mit einer durchschnittlichen Faserlänge in dem Bereich von 1 bis 2 mm enthalten.

9. Verfahren nach irgendeinem vorhergehenden Anspruch, dadurch gekennzeichnet, daß das Verhältnis zwischen dem Fasermaterial und dem Plastikmassen-Matrixmaterial in der erhaltenen Mischung bestimmt wird durch und im wesentlichen entspricht dem Faser/Plastikmassen-Verhältnis des die Ausgangsmischung bildenden Materials.

10. Verfahren nach irgendeinem vorhergehenden Anspruch, dadurch gekennzeichnet, daß das die Ausgangsmischung bildende Material Ausschuß-Prepreg-Schnittabfälle umfaßt.

11. Formmasse, hergestellt nach dem Verfahren nach einem der vorhergehenden Ansprüche, gekennzeichnet durch eine Menge von willkürlich orientierten Fragmenten, die jeweils ein Bündel von wärmehärtbaren Plastik(Kunststoff)-beschichteten Faserfilamenten mit Filamentlängen in dem Bereich von 0,2 bis 10 mm enthalten und wobei das Plastikmassen-Matrixmaterial in einem teilweise ausgehärteten Zustand vorliegt.

12. Masse nach Anspruch 11, dadurch gekennzeichnet, daß die Faserfilamente Kohlenstoff- oder Aramid-Faserfilamente sind.

13. Masse nach Anspruch 11 oder 12, dadurch gekennzeichnet, daß die durchschnittliche Filamentlänge der Faserfilamente in dem Bereich von 1 bis 2 mm liegt.

14. Verfahren zum Formen eines faserverstärkten Plastik(Kunststoff)-Gegenstandes, das umfaßt
das Einbringen einer Formmasse nach Anspruch 11, 12 oder 13 in eine Form, wobei jedes Fragment ein Bündel von mit einem wärmehärtbaren Plastik(Kunststoff)-Material in einem teilweise ausgehärteten Zustand beschichtete Fasern enthält, und
das Erhitzen der Fragmentmasse in der Form unter Druck zum Aushärten des Plastik(Kunststoff)-Materials, wodurch man eine im wesentlichen isotrope faserverstärkte Komponente enthält.

## Revendications

1. Procédé de fabrication d'un composé de moulage qui est un composé de fibres et de matière synthétique, comprenant les étapes où l'on mélange des fibres imprégnées avec un matériau de matrice en matière synthétique, où l'on refroidit le mélange à une température inférieure ou égale à -50°C, caractérisé en ce que le matériau synthétique a la forme d'un matériau synthétique flexible partiellement durci, thermodurcissable et en ce que, après refroidissement, le mélange est moulu ou broyé tant que sa température est encore inférieure ou égale à -50°C pour donner un composé dont les fibres sont plus courtes que celles dudit mélange.

2. Un procédé selon la revendication 1, dans lequel les longueurs'de filaments du composé broyé sont dans la plage de 0,2 à 10 mm.

3. Un procédé selon la revendication 1 ou 2, dans lequel les fibres sont des fibres de carbone.

4. Un procédé selon la revendication 1 ou 2, dans lequel les fibres sont des fibres d'aramide.

5. Un procédé selon l'une quelconque des revendications précédentes, dans lequel le mélange est, un tissu ou un tissu pré-imprégné unidirectionnel, ou des cordes, ou des tresses dont les fibres ont une longueur moyenne de filament supérieure à 10 mm.

6. Un procédé selon l'une quelconque des revendications précédentes, dans lequel le mélange est refroidi en utilisant de l'azote liquide.

7. Un procédé selon la revendication 6, dans lequel le mélange est refroidi au cours du broyage en utilisant de l'azote liquide.

8. Un procédé selon l'une quelconque des revendications qui précèdent, dans lequel le composé broyé comprend des fragments isolés qui comprennent chacun un faisceau de filaments revêtus de matière synthétique dont la longueur moyenne de filament est dans la plage de 1 à 2 mm.

9. Un procédé selon l'une quelconque des revendications qui précèdent, caractérisé en ce que le rapport quantité de matière fibreuse/quantité de matériau de matrice en matière synthétique, dans le composé obtenu, est régi par le rapport de fibre/matière synthétique du matériau formant le mélange de départ et correspond sensiblement à ce dernier.

10. Un procédé selon l'une quelconque des revendications précédentes, dans lequel le matériau formant le mélange de départ comprend des découpes de chutes pré-imprégnées.

11. Un composé de moulage réalisé selon le procédé de l'une des revendications qui précèdent, caractérisé en ce qu'une quantité de morceaux orientés au hasard, comportant chacun un faisceau de matériau fibreux avec des filaments revêtus et avec de la matière synthétique thermodurcissable, filaments dont la longueur est dans la plage de 0,2 mm à 10 mm, le matériau de matrice en matière synthétique étant à l'état partiellement durci.

12. Un composé selon la revendication 11, caractérisé en ce que les filaments fibreux sont des filaments de carbone ou de fibres d'aramide.

13. Un composé selon la revendication 11 ou 12, caractérisé en ce que la longueur moyenne de filament des filaments fibreux est dans la plage de 1 à 2 mm.

14. Un procédé de moulage d'objets en matière plastique à fibres de renforcement, comportant la mise en place d'un composé de moulage dans un moule selon la revendication 11, la revendication 12 ou la revendication 13, chaque morceau comportant un faisceau fibreux revêtu avec un matériau en matière plastique thermodurcissable à l'état partiellement durci, le chauffage de la masse de morceaux dans le moule avec application d'une pression pour durcir le matériau en matière synthétique, ce qui permet de réaliser un composé à fibres renforcées sensiblement isotrope.
